# FASCICULE DE BREVET EUROPEEN

(11) **EP 4 255 344 B1**
(45) Date de publication et mention de la délivrance du brevet: **16.04.2025**
(21) Numéro de dépôt: 21819899.2
(22) Date de dépôt: 03.12.2021
(51) Int. Cl.: A61C 19/00, A61B 90/70

(54) **SYSTÈME ET PROCÉDÉ DE NETTOYAGE ET DÉSINFECTION D'UN INSTRUMENT DENTAIRE**
SYSTEM UND VERFAHREN ZUR REINIGUNG UND STERILISIERUNG EINES ZAHNÄRZTLICHEN INSTRUMENTS
SYSTEM AND METHOD FOR CLEANING AND STERILISING A DENTAL INSTRUMENT

(30) Priorité: 03.12.2020 EP 20211659
(43) Date de publication de la demande: 11.10.2023
(73) Titulaire: Bien-Air Holding SA, 2504 Bienne (CH)
(72) Inventeur: SARCHI, Davide, 8008 Zürich (CH); GALLINA, Marco, 1544 Gletterens (CH); SCHÖNBÄCHLER, Edgar, 2000 Neuchâtel (CH)
(74) Mandataire: BOVARD AG
(86) Numéro de dépôt international: PCT/EP2021/084229
(87) Numéro de publication internationale: WO 2022/117850

(56) Documents cités:
- WO-A1-80/00413
- US-A- 5 723 090
- US-A1- 2004 091 389
- US-B1- 6 368 556

## Description

### Domaine technique de l'invention

La présente invention se rapporte au domaine des instruments et appareils pour les praticiens dans le secteur médical, tels que les dentistes et les chirurgiens dentaires. Plus précisément, elle concerne un système et un procédé de nettoyage et de désinfection par vapeur pour des appareils du type pièce à main ou turbine dentaire.

### État de la technique

Les dentistes, hygiénistes et chirurgiens dentaires, ainsi que d'autres praticiens du secteur notamment de l'endodontie, parodontologie ou de la chirurgie orale ou de l'implantologie ont souvent recours à divers instruments et appareils dont les plus usuels sont les pièces-à-main droites, les contre-angles (appelés ainsi en raison de leur légère courbure) et les turbines à air. Ces appareils comprennent généralement une tête, qui loge un rotor et permet de transférer le mouvement à l'outil de fraisage, découpe ou polissage et qui va dans la bouche du patient, et un manche, c'est-à-dire le composant qui est manipulé par le chirurgien-dentiste. Les pièces-à-main droites et les contre-angles sont raccordées à un moteur (micromoteur) électrique qui fournit l'énergie mécanique nécessaire au mouvement du rotor et donc de l'outil de fraisage, découpe ou polissage. Les turbines à air sont raccordées simplement à un tuyau qui fournit l'air comprimé pour la mise en mouvement du rotor. Le système de couplage d'une turbine à air à un tuyau est d'ailleurs très similaire au système d'accouplement permettant le couplage d'une pièce-à-main ou d'un contre-angle à un moteur.

La partie moteur est montée à la pièce à main via un nez d'accouplement normalisé en vue de transmettre le couple de transmission du moteur ou d'acheminer un flux d'air comprimé mettant en rotation la turbine, et à la fois la pièce à main et la partie moteur comprennent par ailleurs habituellement plusieurs canaux raccordés entre eux, dans lesquels peuvent être acheminés des fluides tels que de l'air comprimé et/ou de l'eau en vue de refroidir le dispositif et respectivement sprayer la zone d'intervention lors de son utilisation. Un exemple d'un agencement de canaux de refroidissement et de spray sont mentionnés par exemple dans le document de brevet EP1768597.

Pour le nettoyage et la désinfection des tels instruments de type pièces-à-main dentaires, les opérations suivantes sont en général réalisées en séquence automatique ou selon paramétrage de l'utilisateur:
- tout d'abord un pré-nettoyage, nettoyage ou rinçage à l'eau froide, tiède ou chaude; suivi d'un
- nettoyage et/ou désinfection à l'aide de produits chimiques; et enfin
- une élimination de débris et/ou séchage via l'application d'air comprimé.

Le document de brevet US5723090 décrit une solution employant une telle méthode pour le nettoyage et la désinfection de pièces à main.

Alternativement ou en complément de la 2^{e} étape de la séquence décrite ci-dessous, on connaît par ailleurs, par exemple du document US2012298151, une méthode de nettoyage et/ou de désinfection utilisant de la vapeur d'eau saturée ou vapeur surchauffée (« over-heated steam »). Lorsque ces deux conditions sont remplies, on se réfère, selon la terminologie anglaise, à une vapeur sèche dite « *dry steam* ».

Une étape de lubrification finale avec huile et éventuellement air ou mélange des deux peut ensuite être prévue.

Le document américain US 6 368 556 B1 décrit une autre solution de nettoyage et de stérilisation pour pièces à main dentaires en autoclave selon laquelle un support de fixation se substituant à la partie moteur est pourvu de canaux dissociés pour nettoyer respectivement le spray via de la vapeur ou de l'air chaud et respectivement les parties rotatives avec de l'huile et de l'air chaud; le processus de stérilisation utilise ici encore de la vapeur sèche à 122° C sous des conditions de pressions de 1.1 Bar dans l'autoclave.

Un inconvénient majeur de ces solutions connues de l'art antérieur est que le nettoyage de l'intérieur des dispositifs n'est pas satisfaisant, car il peut en résulter une accumulation de résidus solides et/ou organiques (par exemple du sang et des protéines) dans les engrenages, roulements à billes etc. Par ailleurs, l'accumulation de résidus rend de moins en moins efficace toute autre procédure de désinfection et/ou de stérilisation, y compris la stérilisation à la vapeur dans un autoclave, et peut aussi provoquer le vieillissement accéléré des dispositifs mécaniques ainsi qu'un risque de surchauffe et de brûlure du patient.

L'inefficacité des procédures de lavage utilisant de l'eau est liée principalement au fait que les gouttes d'eau n'arrivent pas à pénétrer dans les interstices et les zones creuses à l'intérieur des dispositifs ; l'ajout de produits chimiques enzymatiques ou alcalins peut certes accélérer la dissolution des résidus atteignables par les gouttes d'eau mais n'améliore pas l'accès aux zones creuses.

L'inefficacité des procédures de nettoyage alternative utilisant de la vapeur saturée ou surchauffée est liée au fait que la chaleur provoque la dénaturation des protéines, ce qui rend le les saletés et les biofilms plus difficiles à dissoudre et à détacher des surfaces.

Par ailleurs, la désinfection réalisée via une injection de vapeur d'eau saturée ou surchauffée (de type *« dry-steam »)* n'a pas une efficacité universelle, parce que les conditions de haute pression et de haute température de vapeur à l'entrée du dispositif ne sont pas maintenues à travers l'intégralité du dispositif. En effet, le gradient de température entre l'entrée (positionnée généralement à l'arrière du dispositif, au niveau de l'embout remplaçant la partie moteur pour le nettoyage et la désinfection) et la sortie (positionnée dans la tête du dispositif), provoque la condensation de la vapeur sur les surfaces intérieure du dispositif, notamment au niveau de la tête, ce qui limite fortement la capacité bactéricide et fongicides dans les zones du dispositif dentaire les plus contaminées qui se situe précisément au niveau de la tête du dispositif, laquelle est le plus fréquemment amenée en contact la bouche du patient et où l'on est par conséquent plus susceptible de trouver des résidus de sang, de salive et autres colonies bactériennes d'où elles proviennent.

De plus, l'utilisation répétée de vapeur d'eau saturée ou surchauffée à haute pression peut provoquer un vieillissement accéléré des composants mécaniques de la pièce à main.

Le document US 2004/091389 traite enfin d'un dispositif et un procédé de stérilisation d'endoscopes utilisant une application purement séquentielle de vapeur humide suivie d'air comprimé; la durée des cycles de traitement ne peut ainsi pas être optimisée.

Il existe donc un besoin pour des solutions de nettoyage et de désinfections exempts de ces limitations connues.

### Résumé de l'invention

L'invention a pour but de fournir un système et une méthode de nettoyage et de désinfection plus efficace que celles et ceux connus de l'art antérieur.

Un autre but de la présente invention est de fournir un système et une méthode de nettoyage et de désinfection moins agressifs pour les composants mécaniques des pièces traitées comparé aux solutions de l'art antérieur.

Encore un autre but de la présente invention est de fournir une solution minimisant l'usage de produits chimiques habituellement employés pour le nettoyage et la désinfection.

Enfin, un but de la présente invention est de fournir une nouvelle solution permettant de réduire la durée des cycles de nettoyage et de désinfection de l'intérieur de dispositifs.

Selon l'invention, ces buts sont atteints grâce à un système de nettoyage et/ou de désinfection pour outil à usage dentaire ou chirurgical comprenant un générateur de vapeur et une source d'air comprimé, le système de nettoyage et/ou de désinfection comprenant par ailleurs un dispositif de régulation de flux agencé pour injecter de la vapeur humide et de l'air comprimé dans au moins une canalisation de l'outil à usage dentaire ou chirurgical à traiter, caractérisé en ce que le dispositif de régulation de flux est agencé pour injecter au moins partiellement simultanément de la vapeur humide et de l'air comprimé dans au moins une canalisation dudit outil à usage dentaire ou chirurgical à traiter.

Un avantage du nouveau système proposé selon la présente invention est que celui-ci permet d'assurer un nettoyage automatique efficace de l'intérieur des dispositifs dentaires en éviter toute accumulation de biofilms, des résidus de sang et de protéines à l'intérieur de l'outil à traiter.

Un autre avantage du nouveau système proposé selon la présente invention est de pouvoir assurer une désinfection sensiblement homogène de l'ensemble du dispositif dentaire à traiter, y compris de la tête, c'est à dire la partie avant de l'outil.

Un avantage additionnel du nouveau système proposé selon la présente invention est de limiter le vieillissement des composants mécaniques, tels que des roulements à billes ou des joints (O-rings) et de minimiser la corrosion des pièces métalliques par rapport aux procédures traditionnelles de nettoyage/désinfection automatique utilisant des thermo-désinfecteurs ou des désinfecteurs à la vapeur saturée ou surchauffée.

Encore un autre avantage du nouveau système proposé selon la présente invention d'éliminer ou en tous les cas minimiser l'utilisation de produits chimiques pour l'opération de nettoyage et de désinfection

Encore un autre avantage du nouveau système proposé selon la présente invention est de réduire la durée des cycles de nettoyage/désinfection de l'intérieur des dispositifs.

Selon un mode de réalisation préférentiel, le système de nettoyage et/ou de désinfection pour outil à usage dentaire ou chirurgical comprend par ailleurs un support de fixation pour l'outil à usage dentaire ou chirurgical à nettoyer et/ou désinfecter, qui est pourvu d'un nez d'accouplement se substituant à une partie moteur, et le mélange vapeur humide - air peut être injecté directement via ledit nez d'accouplement.

L'avantage d'une telle solution est de permettre une adaptation très simple à des dispositifs de nettoyage et de désinfection existants, en rajoutant simplement un module permettant de générer la vapeur humide et les flux de vapeur souhaités à raccorder à l'arrière de manches de pièces à main.

Selon une variante de ce mode de réalisation préférentiel, le système de nettoyage et/ou de désinfection pour outil à usage dentaire ou chirurgical, le support de fixation est disposé dans une enceinte close, ce qui permet d'y disposer aisément un bac de récupération de l'eau et des déchets extraits du dispositif dentaire ou chirurgical et de les récupérer aisément sans risque de dispersion, d'une part, et d'autre part d'y adjoindre un système de filtrage d'air afin d'éviter l'éjection de contaminants microbiologiques en dehors de l'enceinte. De cette manière, la procédure de nettoyage et de désinfection peut être optimisée d'un point de vue sanitaire.

Selon une autre variante de ce mode de réalisation préférentiel, l'enceinte du système de nettoyage et/ou de désinfection pour outil à usage dentaire ou chirurgical est pourvue d'un système de désinfection par rayons UVC.

L'avantage d'une telle variante est d'assurer la désinfection des surfaces extérieures du dispositif dentaire ou chirurgical ainsi que la désinfection de l'enceinte elle-même, ce qui permet d'éviter le risque de contamination mutuelle entre des dispositifs nettoyés et désinfectés simultanément ou successivement dans une même enceinte.

Selon un autre mode de réalisation préférentiel, le système de nettoyage et/ou de désinfection pour outil à usage dentaire ou chirurgical comprend par ailleurs une interface utilisateur permettant de déterminer au moins un paramètre d'entrée choisi parmi la température, la pression, la fraction de vapeur humide et/ou la fraction d'air comprimé.

L'avantage d'une telle solution est de pouvoir optimiser au mieux le mélange souhaité en fonction des contraintes mécaniques ou électriques du système à nettoyer/désinfecter et/ou de l'acte opératoire réalisé (dentisterie ou chirurgie) avant le nettoyage/désinfection, et donc du besoin réglementaire de réaliser, à la suite du nettoyage/désinfection, une stérilisation ultérieure en autoclave.

Selon un autre mode de réalisation préférentiel, le dispositif de réglage de flux du système de nettoyage et/ou de désinfection pour outil à usage dentaire ou chirurgical proposé comprend une première valve de sortie de vapeur humide et une deuxième valve de sortie d'air comprimé, la deuxième valve étant auto-régulée.

L'avantage d'une telle solution est de permettre de minimiser le nombre de composants du système, ne nécessitant aucune chambre dédiée pour réaliser le mélange, chacun des flux d'air et de vapeur étant amenées séparément jusqu'à l'outil. Un autre avantage est également de maximiser le rendement des cycles de nettoyage et de désinfection, puisqu'il n'est pas nécessaire de réaliser un mélange air-vapeur en équilibre thermodynamique ou quasi-équilibre (c'est-à-dire un état stationnaire dans le temps mais maintenu hors-équilibre par les flux arrivant en continu dans la chambre de mélange) avant l'injection dans l'outil à traiter.

Selon une variante de ce mode de réalisation préférentiel, le dispositif de réglage de flux du système de nettoyage et/ou de désinfection pour outil à usage dentaire ou chirurgical proposé comprend par ailleurs un canal de dérivation raccordé à la deuxième valve, le canal de dérivation étant destiné à acheminer un flux d'air comprimé auxiliaire vers un système d'entraînement pneumatique entraînant en rotation au moins certaines pièces rotatives de l'outil à usage dentaire ou chirurgical à traiter.

L'avantage d'une telle solution est tout d'abord d'optimiser l'efficacité des opérations de nettoyage et de désinfection de parties habituellement en mouvement de l'outil, et ensuite de réaliser cette opération d'entraînement des parties en rotation sans nécessiter de moteur dédié à cet effet, et donc en optimisant parallèlement la complexité et les besoins du dispositif en termes d'alimentation électrique.

Selon un autre mode de réalisation préférentiel, le dispositif de réglage de flux du système de nettoyage et/ou de désinfection pour outil à usage dentaire ou chirurgical proposé comprend par ailleurs une chambre de mélange pour réaliser le mélange vapeur humide - air, qui peut ainsi être délivré via une troisième valve.

L'avantage d'une telle solution est d'imposer des conditions homogènes à l'intérieur du dispositif à nettoyer et à désinfecter, en équilibre ou quasi-équilibre (c'est-à-dire un état stationnaire dans le temps mais maintenu hors-équilibre par les flux arrivant en continu dans la chambre de mélange) thermodynamique, et d'optimiser ainsi les conditions de température, d'humidité et de pression selon les besoins (vitesse d'écoulement, priorisation de l'efficacité du nettoyage sur celle de la désinfection etc.).

La présente invention concerne par ailleurs une méthode de nettoyage et/ou de désinfection d'un outil à usage dentaire ou chirurgical utilisant un système de nettoyage et/ou de désinfection selon l'invention, caractérisée en ce qu'elle comprend une première étape de génération d'un premier flux de vapeur humide et une deuxième étape de génération d'un deuxième flux d'air comprimé, lesdites première étape et deuxième étape étant au moins partiellement simultanées, de manière à injecter au moins partiellement simultanément de la vapeur humide et de l'air comprimé dans au moins une canalisation dudit outil à usage dentaire ou chirurgical à traiter.

Dans la méthode de nettoyage et/ou désinfection il est possible de combiner additionnellement une étape différente où la première étape de génération d'un premier flux de vapeur humide et la deuxième étape de génération d'un deuxième flux d'air comprimé sont ordonnées de façon totalement séquentielle.

Selon l'invention la méthode de nettoyage et/ou de désinfection d'un outil à usage dentaire ou chirurgical utilise toutefois des première et deuxièmes étapes au moins partiellement simultanées. Ainsi, les durées du cycle de traitement sont réduites.

Selon un mode de réalisation préférentiel pour la méthode de nettoyage et/ou de désinfection, la vapeur humide comporte au moins 10% d'eau en phase liquide sous forme de droplets.

Un avantage de cette solution est de définir un bon compromis entre les propriétés de nettoyage et celles de désinfection recherchées, les particules de vapeur pouvant atteindre les zones les plus creuses et inaccessibles et condenser pour dissoudre les résidus et détacher le biofilm, tandis que la concentration d'eau en phase de vapeur et la dimension des particules d'eau ('droplets') à plus faible température permettent de dissoudre le sang et les protéines et transférer vers l'extérieur les résidus de biofilm.

Selon un mode de réalisation préférentiel pour la méthode de nettoyage et/ou de désinfection selon l'invention, la pression efficace du mélange air-vapeur humide est inférieure à 4,5 bars, de manière à ne pas endommager les outils à traiter.

Selon un mode de réalisation préférentiel pour la méthode de nettoyage et/ou de désinfection selon l'invention, la pression partielle de la fraction d'air comprimé est comprise entre 1.5 et 3 bars. Ainsi, il est possible de soutenir un flux homogène de vapeur sans besoin d'augmenter excessivement la pression de la vapeur, grâce à la contribution de l'air.

Selon un mode de réalisation préférentiel pour la méthode de nettoyage et/ou de désinfection selon l'invention, la pression partielle de la fraction de vapeur humide est comprise entre 2 et 4 bars.

L'avantage d'une telle solution est de pouvoir maintenir un écoulement constant à travers du dispositif sans provoquer d'échauffement excessif des surfaces puisque la température de génération de la vapeur augmente avec la pression appliquée.

Selon un mode de réalisation préférentiel pour la méthode de nettoyage et/ou de désinfection selon l'invention, la fraction de vapeur humide du mélange air-vapeur humide est par ailleurs toujours supérieure à 50%, de manière à ne pas devoir générer de pressions et températures trop importantes de vapeur si l'on veut rester dans une plage de valeurs de température suffisantes pour réaliser l'opération de nettoyage et de désinfection - toujours de l'ordre de 100°C - l'effet de l'air étant généralement de refroidir le mélange.

### Brève description des dessins

D'autres avantages et caractéristiques ressortiront plus clairement de la description qui va suivre de modes de réalisation pour la mise en œuvre de l'invention, donnés à titre d'exemple non limitatifs et illustrés sur les dessins annexés, parmi lesquels:
- la figure 1 est un schéma illustrant le fonctionnement d'un dispositif de nettoyage et de désinfection selon un mode de réalisation préférentiel pour l'invention;
- la figure 2 est schéma illustrant le fonctionnement d'un dispositif de nettoyage et de désinfection selon une variante du mode de réalisation préférentiel de la figure 1;
- la figure 3 est un schéma illustrant le fonctionnement d'un dispositif de nettoyage et de désinfection selon un autre mode de réalisation préférentiel pour l'invention;
- la figure 4 est un diagramme illustrant plusieurs séquences possibles pour des cycles de nettoyage et de désinfection , les cycles B et C étant des exemples selon l'invention:
- la figure 5 est un diagramme illustrant la manière dont la combinaison d'eau et de vapeur humide peut avantageusement faire baisser la température d'un mélange employé pour le nettoyage et la désinfection de l'intérieur d'un outil dans le cadre de la présente invention.

### Description de modes de réalisation préférentiels de l'invention

Selon l'état de l'art, la qualité de la vapeur en termes de désinfection a toujours été considérée comme étant inversement proportionnelle à son degré d'humidité, et au contraire directement proportionnelle à la pression appliquée. En effet, l'humidité est susceptible de limiter la quantité de chaleur transmise aux bactéries, tandis qu'un des avantages de la pression appliquée à la vapeur était de pouvoir combiner une action thermique à une action mécanique de nettoyage, liée à l'énergie mécanique d'un fluide à haute pression.

Dans le cadre de la présente invention, on a utilisé les avancées techniques dans la caractérisation de la vapeur humide et des mélanges entre vapeur et air comprimé permettant de mieux connaitre la dimension des droplets d'eau se trouvant dans la vapeur et de mieux maitriser les bilans thermodynamiques des mélanges vapeur-eau-air, et utiliser ainsi des caractéristiques des mélanges vapeur-eau-air, qui avait été toujours considérés comme des défauts jusqu'à présent, pour assurer simultanément :
1. une action de nettoyage mécanique
2. la dissolution chimique des résidus organiques
3. le détachement doux de biofilms des surfaces métalliques
4. des propriétés homogènes de nettoyage et désinfection tout au long du dispositif de la partie arrière à la tête du dispositif, en évitant donc des gradients significatifs en température et humidité.

La faisabilité technique de mélanger la vapeur à l'air était certes connue dans la technologie des autoclaves dans le cadre de processus de stérilisation d'appareils comportant d'ores et déjà un sérum physiologique ou autres; le but dans ce cas était toutefois de pouvoir stériliser l'enveloppe externe de composants pressurisés, nécessitant un environnement à pression constante nécessitant la réalisation d'un vide partiel avant l'introduction de la vapeur. Pour ces systèmes, le mélange air-vapeur est toutefois réalisé dans des conditions quasi-stationnaires et proche de l'équilibre thermodynamique (pression et température similaires), justement pour éviter des gradients excessifs de pression et tout écoulement de flux.

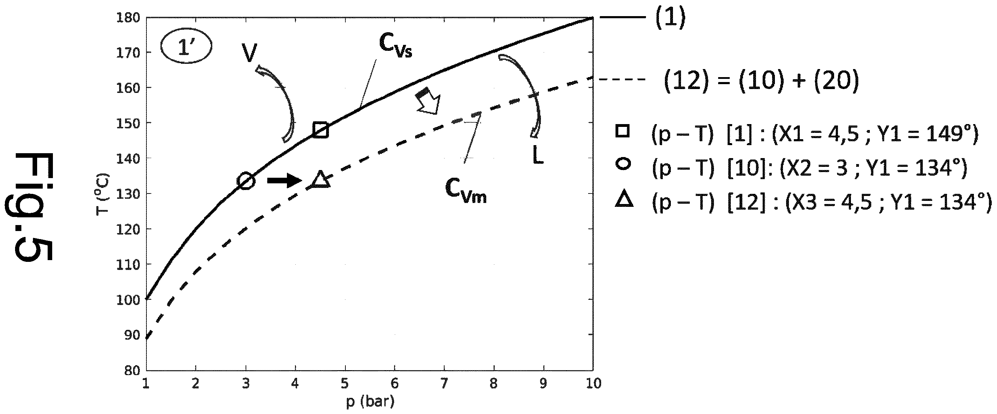

Dans ce qui suit, on montrera comment il est possible de vaincre en particulier le préjugé technique selon lequel l'usage de vapeur humide serait inadaptée à la fois pour un processus de nettoyage et de désinfection de l'intérieur d'une pièce à traiter, et comment il est possible de réaliser un mélange pour optimiser séparément la pression et la température de la vapeur, même si celle-ci est saturée ou proche du niveau de saturation matérialisé par les courbes de pression de vapeur saturantes de la figure 5 discutées ci-après, pour lesquelles il existe une relation stricte entre la pression P et la température T°.

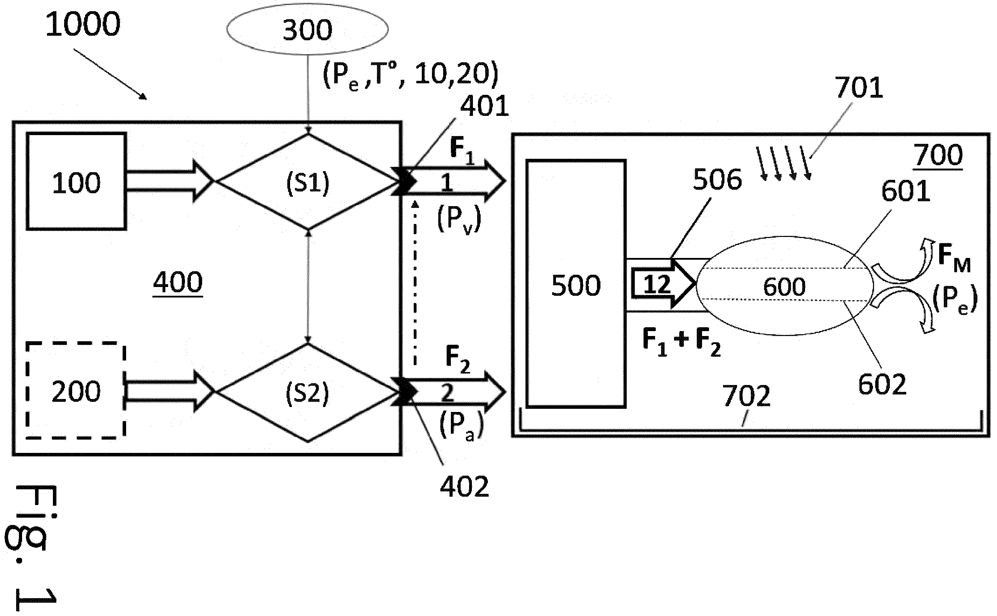

La figure 1 illustre un schéma de fonctionnement d'un système de nettoyage et de désinfection 1000 selon un mode de réalisation préférentiel pour la présente invention, qui vise à injecter de la vapeur humide 1 à l'intérieur de dispositifs dentaires à traiter - les outils 600 - depuis leur partie d'accouplement, c'est-à-dire leur partie arrière, correspondant en principe au manche d'un pièce à main. Selon ce mode de réalisation préférentiel, la partie d'accouplement est formée par un nez d'accouplement 506 standard disposé sur un support de fixation 500 disposé dans une enceinte close 700, c'est-à-dire pouvant être isolée de l'extérieur. Le support de fixation 500 de l'enceinte 700 est ici prévu pour disposer un seul outil à nettoyer et à désinfecter (la rapidité de l'opération réalisée par le système selon l'invention le permet); toutefois on pourrait à titre alternatif pouvoir disposer plusieurs outils à nettoyer et à désinfecter simultanément pour des raisons d'économies d'échelles et si le type de traitement clinique le permet (par exemple si tous les outils ont été utilisés pour traiter le même patient). L'enceinte 700 comporte par ailleurs de préférence un système de désinfection 701 par rayons UVC (sous-ensemble de rayons ultra-violets, de bande spectrale « C »), afin de désinfecter l'extérieur de chacun des outils à traiter et éviter ainsi toute auto-contamination mutuelle. Afin de faciliter le processus de nettoyage de l'enceinte 700 elle-même, un bac de récupération 702 amovible est prévu pour récolter l'eau et les déchets extraits des outils 600. Par ailleurs, un système de filtrage d'air (non illustré) pourrait également être prévu pour éviter toute éjection de contaminant microbiologique en dehors de l'enceinte.

Dans le cadre de la présente invention, on cherche à insérer de l'air comprimé 1 de manière simultanée ou alternative à l'insertion de vapeur humide 2 à l'intérieur des dispositifs dentaires depuis la partie d'accouplement, et le mélange air - vapeur humide 12 ressort ensuite par la tête de l'outil 600, sur la droite de la figure.

Sur le schéma de fonctionnement illustré sur la figure 1, on peut constater que le premier flux F₁ de vapeur humide 1 et deuxième flux F₂ d'air comprimé sont maintenus séparés jusqu'à leur arrivée à la conduite de de délivrance au dispositif, située ici à l'intérieur du nez d'accouplement 506 prévu sur le support de fixation 500. Il n'y a ainsi aucune chambre de mélange des 2 phases, ce qui rend le système de nettoyage et de désinfection 1000 proposé particulièrement simple à mettre œuvre, et peu encombrant. Le premier flux F₁ de vapeur humide 1 s'additionne au deuxième flux F₂ d'air pour générer un flux « combiné » F₁ + F₂ à l'intérieur des canalisations de l'outil 600, constituées par exemple d'un canal central de refroidissement 601 et un ou plusieurs canaux de spray 602 destinés à acheminer de l'air et/ou de l'eau au niveau de la zone de travail à proximité de la tête de l'outil 600. Il n'y a ainsi pas d'équilibre thermodynamique à l'entrée de l'outil 600 à traiter entre le premier flux F₁ de vapeur humide 1 envoyé à une pression partielle dont la valeur Pᵥ qui est potentiellement différente de celle Pₐ et deuxième flux F₂ d'air comprimé, qui est du reste injecté à une température en général proche de la température ambiance (soit aux alentours de 20 degrés), soit bien inférieure à celle de la vapeur, plutôt proche de 100 degrés Celsius. On peut toutefois se rapprocher de cet équilibre au niveau de la tête de l'outil 600, et c'est la raison pour laquelle au niveau des flèches qui en ressortent le flux correspondant au mélange vapeur humide - air 12 est indiqué par la référence F_{M}, et auquel une valeur de pression Pₑ est censée être appliquée. Cette valeur de pression Pₑ correspond à la pression qui peut être définie au niveau d'une interface utilisateur 300 d'un dispositif de réglage de flux 400 situé en amont, et qui définit la pression efficace du mélange, c'est-à-dire tenant compte non seulement des pressions partielles de chacune des phases air-vapeur mais également de leur fraction et volume respectifs dans le mélange.

L'interface utilisateur 300 comprend de préférence un organe de commande tel qu'un bouton ou une interface de saisie telle qu'un écran tactile, et également un module d'affichage pour contrôler la saisie avant de valider le réglage. Selon le mode de réalisation préférentiel décrit, on choisira de préférence une valeur de pression efficace Pₑ qui pourra, en fonction des fractions de vapeur et d'air comprimé dans le mélange, définir une température adéquate pour l'opération de nettoyage et de désinfection. Néanmoins, les paramètres d'entrée ne sont pas limités à une valeur de pression efficace Pₑ mais peuvent également inclure une valeur de température T°, et également ajuster les pourcentages relatifs à la fraction d'air comprimé 20 et la fraction de vapeur humide 10.

Le dispositif de régulation de flux 400 comprend un générateur de vapeur 100 ainsi qu'une source d'air comprimé 200 intégrée ou externe, ainsi qu'une première valve 401 d'où est généré en sortie le premier flux F₁ de vapeur humide 1 et une deuxième valve 402 d'où est généré en sortie le deuxième flux F₂ d'air comprimé. L'ouverture et la fermeture de chacune de ces valves sont effectuées respectivement suite à des étapes de contrôle utilisant par exemple des capteurs associés ou simplement via des temporisateurs programmables, prédéfinis en fonction du paramètre de réglage (pression efficace Pₑ ou température T° ou fraction d'humidité de la vapeur). Sur la figure 1, on se réfère à l'étape de contrôle de la pression de vapeur comme étant l'étape S1, tandis que l'étape de contrôle de la pression d'air comprimé est référencée S2. On peut toutefois constater sur cette figure que selon ce mode de réalisation préférentiel, la deuxième étape de contrôle S2 est en réalité dépendante de la première étape de contrôle S1, car en sélectionnant une valeur de pression efficace Pₑ pour le mélange 12 contenant la vapeur humide et l'air, et pour des proportions d'air et de vapeur humide prédéterminées, la pression partielle d'air Pₐ sera alors automatiquement définie par celle de vapeur Pᵥ. Autrement dit, la deuxième valve 402 sera dans ce cas auto-régulée en fonction de la première valve 401, d'où la flèche matérialisant la liaison entre les deux étapes de contrôle S1,S2 ainsi que la flèche en pointillés indiquant l'alignement de la deuxième valve 402 sur la première valve 401.

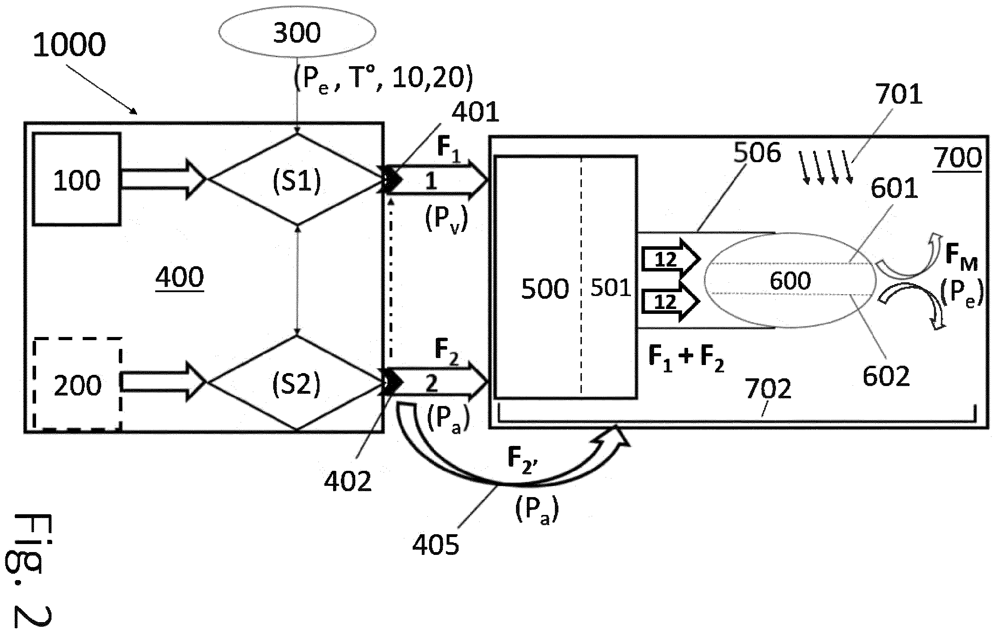

La figure 2 illustre un autre mode de réalisation préférentiel pour le système de nettoyage et/ou de désinfection 1000 pour la présente invention, qui consiste en une variante du premier mode réalisation de la figure 1. Dans un souci de simplification, tous les éléments communs ne seront pas décrits à nouveau en détail; en particulier la gauche de la figure relative au dispositif de régulation de flux 400 qui est totalement identique.

L'amélioration du système de nettoyage et/ou de désinfection 1000 proposé selon cette variante consiste à prévoir un canal de dérivation 405 en sortie de la 2^{e} valve 402, de manière à ce que l'air comprimé 2 puisse être délivré simultanément à deux conduites séparées, l'une étant identique à la précédente pour l'acheminement du deuxième flux d'air comprimé F₂jusqu'à l'outil, et désormais une autre acheminant un flux d'air comprimé auxiliaire F_{2'} destiné à remplir une fonction motrice d'entraînement de certaines parties rotatives de l'outil. Ainsi, alors qu'une première conduite amène le deuxième flux d'air comprimé F₂ conjointement au premier flux de vapeur humide F₁ à l'intérieur du nez d'accouplement 506 et pénétrer dans les conduites de sprays 602 et le canal de refroidissement 601 de l'outil, le flux d'air comprimé auxiliaire F_{2'} généré est envoyé via le canal de dérivation 405 à une pression partielle d'air de valeur Pₐ a priori identique à celle injectée dans le nez d'accouplement 506 du dispositif de fixation de l'outil, en vue d'actionner un dispositif d'entraînement pneumatique 501 relié mécaniquement au support de fixation 500, tel qu'une turbine à hélice. L'action de l'air comprimé permettra de mettre en rotation lente le support nez d'accouplement 506, donc, via la liaison mécanique, la chaine d'engrenage de la pièce à main droite ou du contre-angle. Et lorsque le support 500 comporte une pluralité de nez d'accouplement 506 destinés à recevoir plusieurs outils 600 à traiter, on pourra dans ce cas prévoir des dispositifs d'entraînement 501 dédiés pour chaque nez d'accouplement 506, c'est-à-dire pour actionner en rotation efficacement chacune des chaines d'engrenage de chaque outil 600. Pour ce faire, on pourra soit relier mécaniquement et en parallèle plusieurs chaînes cinématiques à une même hélice, ou alternativement prévoir de dédier des flux d'airs spécifiques à des hélices reliées chacune à un nez d'accouplement 506 correspondant via des dérivations.

Selon les modes de réalisation décrits précédemment, on injecte la vapeur et l'air directement dans la canalisation débouchant dans l'outil 500 à nettoyer/désinfecter en réalisant donc un mélange hors-équilibre thermodynamique. Cette configuration nécessite de moins de composants, mais n'est toutefois optimale en ce qui concerne l'homogénéité du mélange obtenu.

Dans ce qui suit, on décrira un autre mode de réalisation préférentiel impliquant une chambre de mélange séparée de la canalisation débouchant dans l'outil 500 à nettoyer/désinfecter. Dans ce cas, le mélange de vapeur humide - air 12 peut être réalisé proche de l'équilibre thermodynamique ou au moins d'une condition stationnaire de quasi-équilibre (en équilibrant pression et température des composants autour d'un ratio proche de 1, par exemple entre 0.7 et 1.3), ce qui permet éventuellement de récolter une partie de l'eau condensée avant d'injecter dans l'outil non seulement un mélange contenant de l'air et de la vapeur humide (combinaison de vapeur saturé et gouttelettes d'eau en suspension ou 'droplets'), mais aussi au moins partiellement de l'eau sous forme de gouttes qui ne sont pas en suspension . Dans ce cas, la présence d'un système de réglage de la pression du mélange à injecter dans le dispositif est nécessaire, mais permet aussi de régler plus précisément le flux (débit) du mélange prévu pour le nettoyage/désinfection.

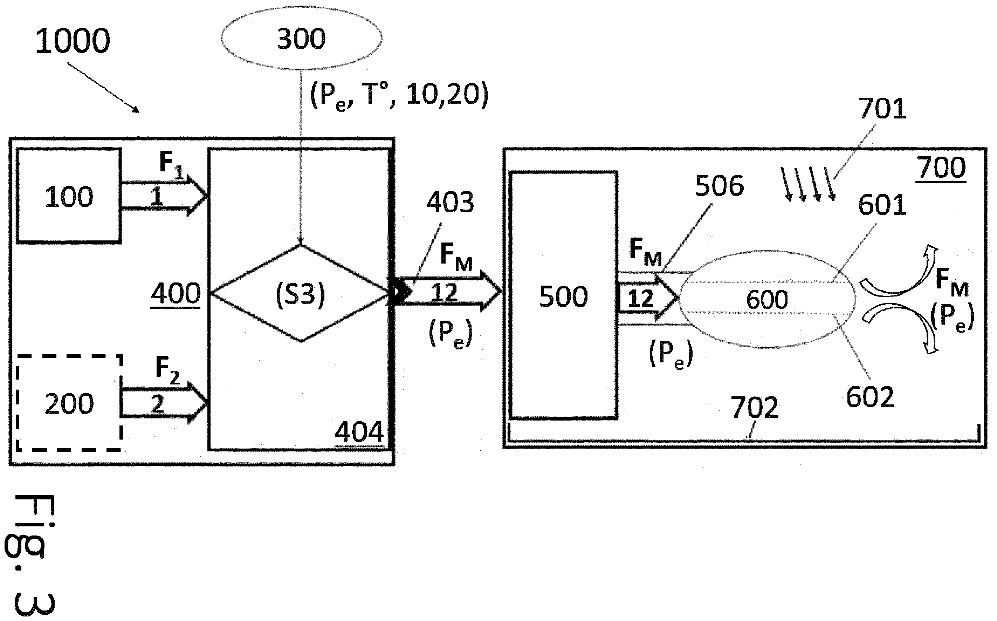

La figure 3 illustre un schéma de fonctionnement pour un tel mode de réalisation ; comme pour la figure 2 précédente, on ne décrira en détail que les éléments qui diffèrent de la figure 1, et on se réfèrera à cette dernière pour tous les éléments communs à celle-ci.

Comme on peut le constater sur la gauche de la figure 3, le générateur de vapeur 100 et la source d'air comprimé 200 intégrée ou externe du dispositif de régulation de flux 400 acheminent un premier flux F₁ de vapeur humide 1 et un deuxième flux F₂ d'air comprimé vers une chambre de mélange 404 des 2 phases. C'est ici la pression efficace Pₑ du mélange vapeur humide - air 12 qui peut être sélectionnée via l'interface utilisateur 300, et un troisième flux F_{M} correspondant au mélange de vapeur humide et d'air 12 est généré en sortie d'une troisième valve 403 suite à une troisième étape de contrôle S3 de la pression de ce mélange vapeur humide - air et/ou de temporisation programmable et définie par le paramètre réglable. Ce troisième flux F_{M} de mélange vapeur humide - air 12 est introduit en équilibre thermodynamique dans les canalisations de l'outil à traiter (canal de refroidissement 601 et canaux de sprays 602) au niveau du nez d'accouplement 506. Ce mode de réalisation est plus facilement contrôlable puisqu'il n'implique plus d'autoréglage de la pression de l'air) et permet d'obtenir un mélange plus homogène à l'intérieur de la conduite d'amenée du fluide au dispositif à traiter, c'est-à-dire l'outil 600, et donc dans l'outil lui-même ; il est cependant évidemment moins compact en termes d'encombrement que le premier mode de réalisation décrit en lien avec la figure 1, en raison du volume de la chambre de mélange.

Bien que non illustrée, on pourra prévoir, pour ce mode de réalisation, une variante similaire à celle de la figure 2 en prévoyant une alimentation directe en air comprimé au support de ou des outils à traiter afin de produire un mouvement de rotation sous l'action pneumatique d'une turbine. Néanmoins, le canal de dérivation devra être aménagé en amont de la chambre de mélange 404.

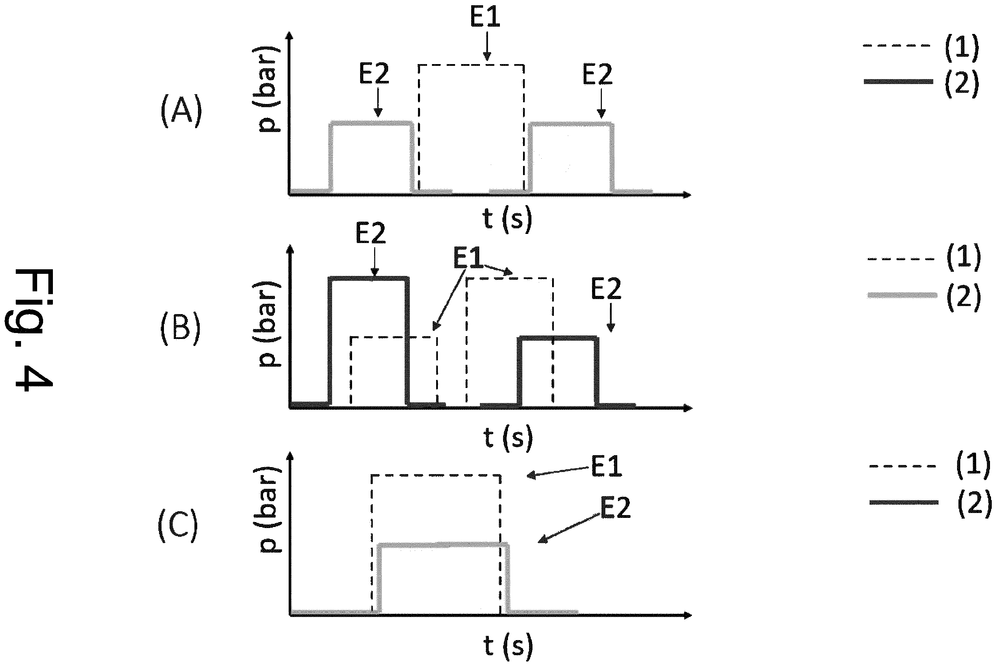

La figure 4 montre 3 exemples non limitatifs de profil de pression de la vapeur humide 1 et de l'air comprimé 2:
- dans l'exemple (A)**,** qui n'est pas le cycle de la méthode de l'invention, mais peut être éventuellement combiné à celle-ci, correspondant à la figure du dessus, l'air 2 et la vapeur humide 1 sont introduits de manière séquentielle, c'est-à-dire l'une à la suite de l'autre sans superposition temporelle : d'abord l'air 2 comprimé pour refroidir le dispositif dentaire/chirurgical, puis la vapeur humide 2 pour nettoyer, puis à nouveau l'air comprimé 2 pour sécher et refroidir à nouveau l'outil.
- dans l'exemple (B)**,** qui un exemple de l'invention, correspondant à la figure du milieu, l'air 2 et la vapeur humide 1 sont introduits de manière non simultanée mais avec une superposition temporelle au moins partielle. La pression de l'air 2 et de la vapeur humide 1, ainsi que le ratio entre elles ne sont pas constantes durant tout le processus; dans le cas décrit ce ratio passe d'une valeur inférieure à 1 à supérieure à 1.
- Dans l'exemple (C)**,** qui un exemple de l'invention, correspondant à la figure du bas, l'air 2 et la vapeur humide 1 sont introduites de manière sensiblement simultanée (90% de superposition temporelle ou plus).

Sur la figure 4 sont ainsi représentées une première étape E1 qui correspond à la génération d'un premier flux F₁ de vapeur humide 1 destiné à être injecté directement ou indirectement dans l'outil à nettoyer et/ou désinfecter, comme ceux illustrés sur les figures 1 à 3, et similairement une deuxième étape E2 de génération d'un deuxième flux F₂ d'air comprimé 2. Ces deux étapes peuvent être ajustées temporellement l'une par rapport à l'autre selon les besoins.

Dans le cadre de l'invention, la vapeur d'eau 1 et l'air 2 sont générés via des flux simultanés, un mélange 12 contenant de la vapeur humide et de l'air est réalisé, dont les propriétés thermodynamiques peuvent être résumées de la manière suivante:
1. P = Pa + Pv : la pression totale est la somme des 2 pressions
2. Pe = P Vv / (Vv + Va) : la pression efficace du mélange vapeur humide - air 12 est réduite par rapport à la pression totale d'un facteur qui dépend des volumes de chacun des 2 fluides (respectivement air et vapeur, leur volumes étant dénotés Va et Vv)

Ces 2 relations ci-dessus montrent qu'il est possible d'obtenir de la vapeur à haute pression avec température réduite. Par exemple, un mélange composé de deux tiers de vapeur à 3 bar (pression absolue) et d'un tiers d'air à 1.5 bar (absolue) produit un mélange à 4.5 bar à environ 134°C, ce qui est idéal pour la désinfection sans induire un échauffement excessif du dispositif à désinfecter, alors que la vapeur pure à 4.5 bar serait proche de 150°C, donc potentiellement néfaste pour certains composants mécaniques.

Cet exemple est représenté en Fig. 5, illustrant deux courbes de pression de vapeur saturante respectivement pour de la vapeur 1 saturée, et respectivement un mélange 12 contant de la vapeur saturée et de l'air comprimé. Pour chacune de ces courbes, il existe une relation stricte entre la pression P et la température T° correspondant à une ligne de démarcation correspondant au changement de phase entre la phase liquide « L » et la phase vapeur « V »:
- La courbe C_{Vs} relative à la vapeur saturée pure démarre à 100°C sous pression atmosphérique de 1 bar, pour atteindre un peu moins de 150°C sous une pression de 4.5 bar [point de coordonnées (X1,Y1) sur cette courbe matérialisé par un carré]
- La courbe C_{Vm} relative à un mélange comprenant une fraction de vapeur humide 10 égale à 66,6% et une fraction d'air comprimé 20 également à 33,3% pour former un mélange 12 vapeur-humide - air démarre quant à elle aux alentour de 90°C sous pression atmosphérique, et n'atteint que 134°C sous une pression de 4.5 bars [point de coordonnées (X3,Y3) sur cette courbe matérialisé par un triangle], cette température correspondant d'une vapeur saturée sous une pression de 3 bar [point de coordonnées (X2,Y2) sur la courbe C_{Vs} précédente matérialisé par un rond]

On peut ainsi aisément constater que la courbe de pression-température d'un mélange avec 33% d'air 2 comprimé est environ 15°C en dessous de la courbe de la vapeur saturée pure C_{Vs}. Le mélange de vapeur humide à 3 bar et de l'air à 1.5 bar permet ainsi d'augmenter la pression à 4.5 bar (et donc de maximiser l'effet mécanique du nettoyage), sans avoir besoin d'augmenter excessivement la température: une pression équivalente ne serait pas atteignable avec de la vapeur pure sans dépasser largement la température de stérilisation / désinfection habituelle (149°C vs 134°C). La flèche pointant de la courbe C_{Vs} vers la courbe C_{Vm} inclinée substantiellement vers le bas permet d'illustrer comment le décalage de la relation p-T° entre ces deux courbes permet d'obtenir cette réduction de température bénéfique.

On comprendra de ce qui précède qu'en utilisant cette technique de « dilution » de vapeur humide avec de l'air, il serait possible d'obtenir des mélanges vapeur humide - air 12 pour le nettoyage se rapprochant de 100°C en augmentant la pression de manière correspondant et en augmentant encore la contribution de l'air dans le mélange, c'est-à-dire en augmentant le pourcentage de la fraction d'air comprimé 20. Toutefois, afin de rester dans des conditions de travail où la température reste suffisamment élevée pour conserver des propriétés désinfectantes efficaces, mais sans toutefois générer de pression trop élevée pouvant endommager les outils à traiter, on privilégiera une fraction de vapeur humide 20 toujours supérieure à 50%. Pour les mêmes raisons, on privilégiera des valeurs de pression efficace Pₑ du mélange inférieures à 4.5 bar, et une pression partielle correspondant à la fraction de vapeur humide 10 comme étant toujours inférieure à 4 bar. Selon un mode de réalisation particulièrement préféré, le taux de fraction de vapeur humide 20 est compris entre 50% et 66% du mélange total.

Par ailleurs, afin de maximiser les propriétés nettoyantes du mélange air-vapeur humide 12 obtenu, pour pourra rechercher à obtenir de la vapeur humide 1 comportant au moins 10% d'eau en phase liquide sous forme de droplets. Autrement dit, on se situera légèrement en dessous de la courbe définissant la pression de vapeur saturante du mélange 12, c'est-à-dire du côté où la phase liquide est censée redevenir prépondérante. La flèche pointant vers le bas et le « L » matérialise la direction du changement de phase vers la phase liquide, tandis que la flèche pointant vers le haut et le « V » matérialise la direction de changement de phase opposée vers la phase vapeur, la vapeur sèche 1' correspondant en réalité à l'intégralité de la partie du diagramme située au-dessus de la courbe de pression de vapeur saturante.

Dans le cadre de la présente invention, suite à la phase de nettoyage/désinfection, il serait encore possible de rajouter une étape de désinfection pure, réalisé avec l'injection de vapeur sèche surchauffée, ou d'un mélange de vapeur sèche surchauffée et d'air comprimé, les propriétés de ce mélange étant définies selon la procédure décrite en figure 5. En fait, à la suite d'un nettoyage efficace, l'efficacité de désinfection de la vapeur sèche surchauffée serait fortement améliorée, ce qui permet d'utiliser des valeurs de pression modérées, typiquement inférieures à 4.5 bar et avantageusement inférieure à 3.5 bar, pour mieux préserver les outils dentaires ou chirurgicaux à traiter.

Avantageusement, le dispositif de nettoyage et désinfection peut être équipé d'un réservoir d'eau intégré, à remplir avec eau distillée, déminéralisée, ou du robinet si la dureté le permet. Dans ce cas, le volume optimum du réservoir d'eau est compris entre 0.3 l et 0.7 l, pour optimiser le nombre de nettoyages/désinfections réalisables avant de remplir le réservoir (un remplissage par semaine, en raison de 30 nettoyages/désinfections par jour étant optimal) et minimiser les dimensions du dispositif.

La description détaillée du fonctionnement du nouveau système proposé selon un mode de réalisation préférentiel a été faite dans le cas d'un nez d'accouplement pour contre-angle ou pièces-à-main droites. Dans le cas où l'outil à nettoyer et à désinfecter est une turbine, les seules différences sont relatives à la géométrie du nez d'accouplement, qui prendra la forme d'un accouplement turbine, et dans le moyen de mise en mouvement du rotor qui est dans ce cas mis en mouvement simplement par le passage du fluide sous pression (la vapeur humide, l'air comprimé ou le mélange des deux fluides).

On comprendra par conséquent de ce qui précède que l'outil à usage dentaire chirurgical 600 à traiter peut indifféremment consister en un contre-angle, une pièce-à-main droite, ou une turbine.

Par ailleurs, dans le cadre de la présente invention, on s'est référé à un générateur de vapeur 100 pour générer un premier flux de vapeur humide F₁ à injecter, directement ou indirectement, c'est-à-dire via un mélange préalable ou non avec un deuxième flux d'air F₂, dans l'outil 600 à traiter. On comprendra qu'un tel générateur de vapeur 100 ne se limite pas à un générateur de vapeur humide saturée, mais pourra également inclure un générateur de vapeur au moins partiellement sèche, pour autant que le flux de vapeur résultant injecté dans l'outil, ou ressortant de la tête de l'outil traité, contienne de la vapeur humide, l'idée sous-jacente de l'invention étant précisément d'utiliser de la vapeur humide pour l'opération de nettoyage et/ou de désinfection, et non plus uniquement de la vapeur sèche.

## Revendications

1. Système de nettoyage et/ou de désinfection (1000) pour outil à usage dentaire ou chirurgical (600) comprenant un générateur de vapeur (100) et une source d'air comprimé (200), ledit système de nettoyage et/ou de désinfection (1000) comprenant par ailleurs un dispositif de régulation de flux (400) agencé pour injecter de la vapeur humide (1) et de l'air comprimé (2) dans au moins une canalisation dudit outil à usage dentaire ou chirurgical (600) à traiter, **caractérisé en ce que** le dispositif de régulation de flux (400) est agencé pour injecter au moins partiellement simultanément de la vapeur humide (1) et de l'air comprimé (2) dans au moins une canalisation dudit outil à usage dentaire ou chirurgical (600) à traiter.

2. Système de nettoyage et/ou de désinfection (1000) pour outil à usage dentaire ou chirurgical (600) selon la revendication 1, **caractérisé en ce qu'**il comprend un support de fixation (500) pour ledit outil à usage dentaire ou chirurgical (600) à nettoyer et/ou désinfecter, ledit support de fixation (500) étant pourvu d'un nez d'accouplement (506) se substituant à une partie moteur, et ledit mélange vapeur humide - air (12) pouvant être injecté directement via ledit nez d'accouplement (506).

3. Système de nettoyage et/ou de désinfection (1000) pour outil à usage dentaire ou chirurgical (600) selon la revendication 2, **caractérisé en ce que** ledit support de fixation (500) est disposé dans une enceinte (700) close.

4. Système de nettoyage et/ou de désinfection (1000) pour outil à usage dentaire ou chirurgical (600) selon la revendication 3, ladite enceinte étant pourvue d'un système de désinfection par rayons UVC (701).

5. Système de nettoyage et/ou de désinfection (1000) pour outil à usage dentaire ou chirurgical (600) selon l'une des revendications précédentes, **caractérisé en ce qu'**il comprend par ailleurs une interface utilisateur (300) permettant de déterminer au moins un paramètre d'entrée choisi parmi la température, la pression, la fraction de vapeur humide (10) et/ou la fraction d'air comprimé (20).

6. Système de nettoyage et/ou de désinfection (1000) pour outil à usage dentaire ou chirurgical (600) selon l'une des revendications précédentes, **caractérisé en ce que** le dispositif de réglage de flux (400) comprend une première valve (401) de sortie de vapeur humide (1) et une deuxième valve (402) de sortie d'air comprimé (2), ladite deuxième valve (402) étant auto-régulée.

7. Système de nettoyage et/ou de désinfection (1000) pour outil à usage dentaire ou chirurgical (600) selon la revendication 6, **caractérisé en ce que** le dispositif de réglage de flux (400) comprend par ailleurs un canal de dérivation (405) raccordé à ladite deuxième valve (402), ledit canal de dérivation étant destiné à acheminer un flux d'air comprimé auxiliaire (F₂') vers un système d'entraînement pneumatique (501) entraînant en rotation au moins certaines pièces rotatives dudit outil à usage dentaire ou chirurgical (600).

8. Système de nettoyage et/ou de désinfection (1000) pour outil à usage dentaire ou chirurgical (600) selon l'une des revendications 1 à 5, **caractérisé en ce que** le dispositif de réglage de flux (400) comprend par ailleurs une chambre de mélange (404) pour réaliser un mélange vapeur humide - air (12), lequel peut être délivré via une troisième valve (403).

9. Méthode de nettoyage et/ou de désinfection d'un outil à usage dentaire ou chirurgical (600) utilisant un système de nettoyage et/ou de désinfection (1000) selon l'une des revendications 1 à 8 précédentes, **caractérisée en ce qu'**elle comprend une première étape (E1) de génération d'un premier flux (F₁) de vapeur humide (1) et une deuxième étape (E2) de génération d'un deuxième flux (F₂) d'air comprimé (2), lesdites première étape (E1) et deuxième étape (E2) étant au moins partiellement simultanées, de manière à injecter au moins partiellement simultanément de la vapeur humide (1) et de l'air comprimé (2) dans au moins une canalisation dudit outil à usage dentaire ou chirurgical (600) à traiter.

10. Méthode de nettoyage et/ou de désinfection d'un outil à usage dentaire ou chirurgical (600) selon la revendication 9, ladite vapeur humide (1) comportant au moins 10% d'eau en phase liquide sous forme de droplets.

11. Méthode de nettoyage et/ou de désinfection d'un outil à usage dentaire ou chirurgical (600) selon l'une des revendications 9 ou 10, la pression efficace dudit mélange air-vapeur humide (12) étant inférieure à 4,5 bar.

12. Méthode de nettoyage et/ou de désinfection d'un outil à usage dentaire ou chirurgical (600) selon l'une des revendications 9 à 11, **caractérisée en ce que** la pression partielle de la fraction d'air comprimé (20) est comprise entre 1.5 et 3 bar.

13. Méthode de nettoyage et/ou de désinfection d'un outil à usage dentaire ou chirurgical (600) selon l'une des revendications 9 à 12, **caractérisée en ce que** la pression partielle de la fraction de vapeur humide (10) est comprise entre 2 et 4 bar.

14. Méthode de nettoyage et/ou de désinfection d'un outil à usage dentaire ou chirurgical (600) selon l'une des revendications 9 à 13, **caractérisée en ce que** la fraction de vapeur humide (10) dudit mélange air-vapeur humide (12) est supérieure à 50%.

## Patentansprüche

1. System (1000) zur Reinigung und/oder Desinfektion eines Werkzeugs (600) zur zahnärztlichen oder chirurgischen Verwendung, das einen Dampferzeuger (100) und eine Druckluftquelle (200) umfasst, wobei das System (1000) zur Reinigung und/oder Desinfektion außerdem eine Flussregulierungsvorrichtung (400) umfasst, die eingerichtet ist, Nassdampf (1) und Druckluft (2) in mindestens einen Kanal des zu behandelnden einleitet, **dadurch gekennzeichnet, dass** die Flussregulierungsvorrichtung (400) derart angeordnet ist, zumindest teilweise gleichzeitig Nassdampf (1) und Druckluft (2) in mindestens einen Kanal des zu behandelnden Werkzeugs (600) zur zahnärztlichen oder chirurgischen Verwendung einzuleiten.

2. System (1000) zur Reinigung und/oder Desinfektion eines Werkzeugs (600) zur zahnärztlichen oder chirurgischen Verwendung nach Anspruch 1, **dadurch gekennzeichnet, dass** es eine Befestigung (500) für das zu reinigende und/oder zu desinfizierende Werkzeug (600) zur zahnärztlichen oder chirurgischen Verwendung umfasst, wobei die Befestigung (500) mit einer Kupplungsnase (506) als Ersatz für ein Motorteil versehen ist, und wobei das Nassdampf-Luft-Gemisch (12) direkt über die Kupplungsnase (506) injizierbar ist.

3. System (1000) zur Reinigung und/oder Desinfektion eines Werkzeugs (600) zur zahnärztlichen oder chirurgischen Verwendung nach Anspruch 2, **dadurch gekennzeichnet, dass** die Befestigung (500) in einem geschlossenen Gehäuse (700) angeordnet ist.

4. System (1000) zur Reinigung und/oder Desinfektion eines Werkzeugs (600) zur zahnärztlichen oder chirurgischen Verwendung nach Anspruch 3, wobei das Gehäuse mit einem UVC-Desinfektionssystem (701) versehen ist

5. System (1000) zur Reinigung und/oder Desinfektion eines Werkzeugs (600) zur zahnärztlichen oder chirurgischen Verwendung nach einem der vorhergehenden Ansprüche, **dadurch gekennzeichnet, dass** es ferner eine Benutzerschnittstelle (300) umfasst, die es ermöglicht, mindestens einen Eingangsparameter zu bestimmen, der aus Temperatur, Druck, Nassdampfanteil (10) und/oder Druckluftanteil (20) ausgewählt ist

6. System (1000) zur Reinigung und/oder Desinfektion eines Werkzeugs (600) zur zahnärztlichen oder chirurgischen Verwendung nach einem der vorhergehenden Ansprüche, **dadurch gekennzeichnet, dass** die Flussregulierungsvorrichtung (400) ein erstes Ventil (401) für den Austritt von Nassdampf (1) und ein zweites Ventil (402) für den Austritt von Druckluft (2) umfasst, wobei das zweite Ventil (402) selbstregulierend ist.

7. System (1000) zur Reinigung und/oder Desinfektion eines Werkzeugs (600) zur zahnärztlichen oder chirurgischen Verwendung nach Anspruch 6, **dadurch gekennzeichnet, dass** die Flussregulierungsvorrichtung (400) außerdem einen Bypasskanal (405) umfasst, der mit dem zweiten Ventil (402) verbunden ist, wobei der Bypasskanal dazu bestimmt ist, einen Druckluft-Hilfsstrom (F2') an ein pneumatischen Antriebssystem (501) zu leiten, das mindestens einige rotierende Teile Werkzeugs (600) zur zahnärztlichen oder chirurgischen Verwendung in Rotation versetzt.

8. System (1000) zur Reinigung und/oder Desinfektion eines Werkzeugs (600) zur zahnärztlichen oder chirurgischen Verwendung nach einem der Ansprüche 1 bis 5, **dadurch gekennzeichnet, dass** die Flussreguliervorrichtung (400) zudem eine Mischkammer (404) zur Herstellung eines Nassdampf-Luft-Gemischs (12) umfasst, das über ein drittes Ventil (403) abgegeben werden kann.

9. Verfahren zur Reinigung und/oder Desinfektion eines Werkzeugs (600) zur zahnärztlichen oder chirurgischen Verwendung mittels eines Systems (1000) zur Reinigung und/oder Desinfektion nach einem der vorhergehenden Ansprüche 1 bis 8, **dadurch gekennzeichnet, dass** es einen ersten Schritt (E1) zur Erzeugung eines ersten Stroms (F1) von Nassdampf (1) und einen zweiten Schritt (E2) zur Erzeugung eines zweiten Stroms (F2) von Druckluft (2) umfasst, wobei der erste Schritt (E1) und der zweite Schritt (E2) zumindest teilweise gleichzeitig erfolgen, um zumindest teilweise gleichzeitig Nassdampf (1) und Druckluft (2) in mindestens einen Kanal des zu behandelnden Werkzeugs (600) zur zahnärztlichen oder chirurgischen Verwendung einzuleiten.

10. Verfahren zur Reinigung und/oder Desinfektion eines Werkzeugs (600) zur zahnärztlichen oder chirurgischen Verwendung nach Anspruch 9, wobei der Nassdampf (1) mindestens 10% Wasser in flüssiger Phase in Form von Tropfen enthält.

11. Verfahren zur Reinigung und/oder Desinfektion eines Werkzeugs (600) zur zahnärztlichen oder chirurgischen Verwendung nach einem der Ansprüche 9 oder 10, wobei der effektive Druck des Nassdampf-Luft-Gemischs (12) weniger als 4.5 bar beträgt.

12. Verfahren zur Reinigung und/oder Desinfektion eines Werkzeugs (600) zur zahnärztlichen oder chirurgischen Verwendung nach einem der Ansprüche 9 bis 11, **dadurch gekennzeichnet, dass** der Partialdruck des Druckluftanteils (20) zwischen 1.5 und 3 bar liegt.

13. Verfahren zur Reinigung und/oder Desinfektion eines Werkzeugs (600) zur zahnärztlichen oder chirurgischen Verwendung nach einem der Ansprüche 9 bis 12, **dadurch gekennzeichnet, dass** der Partialdruck des Nassdampfanteils (10) zwischen 2 und 4 bar liegt.

14. Verfahren zur Reinigung und/oder Desinfektion eines Werkzeugs (600) zur zahnärztlichen oder chirurgischen Verwendung nach einem der Ansprüche 9 bis 13, **dadurch gekennzeichnet, dass** der Nassdampfanteil (10) des Nassdampf-Luft-Gemischs (12) größer als 50% ist.

## Claims

1. System (1000) for cleaning and/or disinfecting a tool (600) for dental or surgical use, comprising a steam generator (100) and a compressed air source (200), said cleaning and/or disinfecting system (1000) further comprising a flow regulating device (400) designed to inject wet steam (1) and compressed air (2) into at least one channel of the tool (600) for dental or surgical use that is to be treated, **characterized in that** the flow regulating device (400) is arranged to inject wet steam (1) and compressed air (2) at least partially simultaneously into at least one channel of the tool (600) for dental or surgical use that is to be treated.

2. System (1000) for cleaning and/or disinfecting a tool (600) for dental or surgical use according to claim 1, **characterized in that** it comprises a fixing support (500) for said tool (600) for dental or surgical use to be cleaned and/or disinfected, said fixing support (500) being provided with a coupling nose (506) as a substitute for a motor part, and said wet steam-air mixture (12) being injectable directly via said coupling nose (506).

3. System (1000) for cleaning and/or disinfecting a tool (600) for dental or surgical use according to claim 2, **characterized in that** said fixing support (500) is disposed in a closed enclosure (700).

4. System (1000) for cleaning and/or disinfecting a tool (600) for dental or surgical use according to claim 3, said enclosure being provided with a disinfection system (701) with UVC rays.

5. System (1000) for cleaning and/or disinfecting a tool (600) for dental or surgical use according to one of the preceding claims, **characterized in that** it further comprises a user interface (300) making it possible to determine at least one input parameter selected from among temperature, pressure, wet steam fraction (10) and/or compressed air fraction (20).

6. System (1000) for cleaning and/or disinfecting a tool (600) for dental or surgical use according to one of the preceding claims, **characterized in that** the flow regulating device (400) comprises a first valve (401) for the outlet of wet steam (1) and a second valve (402) for the outlet of compressed air (2), said second valve (402) being self-regulating.

7. System (1000) for cleaning and/or disinfecting a tool (600) for dental or surgical use according to claim 6, **characterized in that** the flow regulating device (400) further comprises a bypass channel (405) connected to said second valve (402), said bypass channel being intended to convey an auxiliary compressed air flow (F2') to a pneumatic drive system (501) driving in rotation at least some of the rotating parts of said tool (600) for dental or surgical use.

8. System (1000) for cleaning and/or disinfecting a tool (600) for dental or surgical use according to one of the claims 1 to 5, **characterized in that** the flow regulating device (400) further comprises a mixing chamber (404) for making a wet steam-air mixture (12), which is able to be delivered via a third valve (403).

9. Method for cleaning and/or disinfecting a tool (600) for dental or surgical use using a system (1000) for cleaning and/or disinfecting according to one of the preceding claims 1 to 8, **characterized in that** it comprises a first step (E1) of generating a first flow (F1) of wet steam (1) and a second step (E2) of generating a second flow (F2) of compressed air (2), said first step (E1) and second step (E2) being at least partially simultaneous to inject at least partially simultaneously wet steam (1) and compressed air (2) into at least one channel of said tool (600) for dental or surgical use that is to be treated.

10. Method for cleaning and/or disinfecting a tool (600) for dental or surgical use according to claim 9, said wet steam (1) comprising at least 10% water in liquid phase in the form of droplets.

11. Method for cleaning and/or disinfecting a tool (600) for dental or surgical use according to one of the claims 9 or 10, the effective pressure of said wet air-steam mixture (12) being less than 4.5 bar.

12. Method for cleaning and/or disinfecting a tool (600) for dental or surgical use according to one of the claims 9 to 11 **characterized in that** the partial pressure of the compressed air fraction (20) is between 1.5 and 3 bar.

13. Method for cleaning and/or disinfecting a tool (600) for dental or surgical use according to one of the claims 9 to 12, **characterized in that** the partial pressure of the wet steam fraction (10) is between 2 and 4 bar.

14. Method for cleaning and/or disinfecting a tool (600) for dental or surgical use according to one of the claims 9 to 13, **characterized in that** the wet steam fraction (10) of said wet air-steam mixture (12) is greater than 50%.
